Europäisches Patentamt

European Patent Office

Office européen des brevets

(11) Publication number: **0 402 450 B1**

## EUROPEAN PATENT SPECIFICATION

(45) Date of publication of patent specification: **04.05.94**

(21) Application number: **90901275.9**

(22) Date of filing: **21.11.89**

(86) International application number:
**PCT/US89/05352**

(87) International publication number:
**WO 90/05459 (31.05.90 90/12)**

(51) Int. Cl.5: **A23C 9/123**, C12N 15/01,
C12N 15/56, C12N 15/52,
//C12N1/20,C12R1:225

(54) PRODUCTION OF FERMENTED FOOD PRODUCTS.

(30) Priority: **21.11.88 US 274582**

(43) Date of publication of application:
**19.12.90 Bulletin 90/51**

(45) Publication of the grant of the patent:
**04.05.94 Bulletin 94/18**

(84) Designated Contracting States:
**AT BE CH DE ES FR GB IT LI LU NL SE**

(56) References cited:
**US-A- 4 734 361**

(73) Proprietor: **GENENCOR, INC.**
**180 Kimball Way**
**South San Francisco California 94080(US)**

(72) Inventor: **MAINZER, Stanley, E.**
**1337 DeSoto Ave.**
**Burlingame, CA 94010(US)**
Inventor: **YOAST, Sienna**
**Star Route 2**
**Box 410**
**La Honda, CA 94020(US)**

Inventor: **ADAMS, Robin, M.**
**1412 DeSoto Avenue**
**Burlingame CA 94010(US)**
Inventor: **PALOMBELLA, Tony, L.**
**2727 Folsom St., No. 327**
**Boulder, CO 80302(US)**
Inventor: **SCHMIDT, Brian, F.**
**485 Laurel Avenue, 4**
**Half Moon Bay, CA 94019(US)**

(74) Representative: **Stuart, Ian Alexander et al**
**MEWBURN ELLIS**
**York House**
**23 Kingsway**
**London WC2B 6HP (GB)**

**Description**

The present invention relates to novel methods of making a fermented food product, especially fermented dairy products. Further, the invention relates to novel mutant strains of Lactobacillus bulgaricus - (L. bulgaricus) and methods of making them which are useful in the preparation of yogurt and other fermented food products. The invention also relates to novel mutant genes and organisms transformed with such genes.

A variety of food products are available worldwide which depend on active bacterial cultures in the final form of the food product for flavor, preservation of quality, claimed health benefits and/or pH. Examples are fermented vegetable products, such as sauerkraut from cabbage and pickles from cucumbers; fermented fish products such as fish paste or burongdalog; fermented seeds such as coffee or cocoa beans; fermented starch-rich food products; fermented meat products, fermented cassava; or fermented fruit juices. Particularly important are fermented dairy products such as yogurt, sour cream, creme fraché; buttermilk, and the like. These fermented dairy products depend, to a certain degree, on organisms which metabolize lactose to produce flavor and lower the pH to preserve the food. These products can be especially important those people with lactose intolerance; i.e., the inability or difficulty in metabolizing lactose.

Yogurt is an extremely popular fermented dairy product. Microbiologically, yogurt may be defined in the United States and many other countries as the end product of a controlled fermentation of milk with a mixture of Streptococcus thermophilus (S. thermophilus) and L. bulgaricus. The fermentation is carried out within a temperature range of 35 - 45°C with about 42 - 45°C being preferred. Early in the incubation, S. thermophilus grows rapidly dominating the fermentation and lowering the oxidation-reduction potential of the system. L. bulgaricus grows slowly during the early fermentation but liberates sufficient amounts of peptides and amino acids to stimulate growth of S. thermophilus which results in moderate production of lactic acid, acetic acid, acetaldehyde, diacetyl and formic acid. When the pH of the fermentation mix is sufficiently lowered to around 5.5, the rapid growth of S. thermophilus is arrested and the growth of L. bulgaricus is favored. Depletion of oxygen from the system and the availability of formate is believed to stimulate such growth. The major portion of lactic acid and acetaldehyde necessary for the characteristic flavor of yogurt is contributed by the L. bulgaricus which has been aided by the initial activity of the S. thermophilus component. When the pH drops further to about 5.0 or less the product is cooled to about 10°C or less for storage. Although the rate of production of lactic acid is diminished under the normal storage conditions of yogurt (4 - 10°C), production of lactic acid continues to such a degree that the yogurt product is, depending on strain or process, rendered unpalatable (sour) after 3 or 4 weeks. Attempts to further slow or arrest the production of lactic acid during storage while maintaining viable organisms have been largely unsuccessful. Preservatives have been tried but have been undesirable side effect of affecting the viability or killing the yogurt organism. Attempts at mutating the organism directly; e.g., through chemical or other mutagenesis techniques have not only produced organisms with decreased lactic acid production at lower temperature and pH, but organisms also showing a proportional decrease in lactic acid production at fermentation temperatures and a decreased growth, thus producing an unsatisfactory yogurt product or products which are limited to the strain which has been mutated (with an unknown effect), which can not be easily transferred to new starters (see U.S. Patent 4,734,361 to Meiji Milk Products wherein a method is described for isolating a naturally-occuring varient of Lactobacillus bulgaricus). This temperature-sensitive organism produces less than .1% lactic acid at 10°C for 7 days. This method is dependent on a strain being present which matches the description of the invention, and, to date, only one such natural isolate is known to exist, 0LL 1074. Currently no cultures, especially of L. bulgaricus, are available which combine the desired needs of texture and taste while maintaining the extended lowered metabolism under storage temperature, have the site of mutation defined and are transferable to new starters.

It would be useful to construct a L. bulgaricus which exhibited both decreased production of lactic acid at the storage temperature of yogurt yet still retained acceptable levels of activity under production conditions of yogurt where the mutation is defined and which could be transferable to new starters starting from any single strain of L. bulgaricus and remains viable in the yogurt product. It would also be useful to have such organism produce less acid at temperatures below 20°C or at pH 5.5 or lower in order to give more flexibility during production and handling after production. Furthermore, it would aid in the manufacture of fermented products to have the fermentation slow or stop after reaching a certain range of temperature and/or pH rather than using a quick refrigeration to decrease the rate of acid formation.

It is an object of this invention to provide novel organisms and methods for obtaining them where such organisms are for use in fermented food products where the organism exhibits decreased metabolism beyond what is normal under storage temperatures of the fermented food products, yet retains its activity and viability under production conditions of the fermented food product.

It is further an object of the invention to provide novel fermented food products containing viable organisms which, under normal storage conditions of the fermented food products, develop excess acidity or off flavors more slowly than previously fermented food products containing viable organisms.

It is also an object of the invention to provide methods of producing fermented food products.

It is also an object of this invention to provide novel organisms which exhibit decreased production of lactic acid at the storage conditions of yogurt or other food products, yet essentially the novel organisms retain their activity and viability under production and storage conditions. It is a further object of this invention to provide a method of obtaining such organisms.

Even further it is an object of the invention to provide a method of producing yogurt or other food products which simplifies both the processing and handling of the product. It is yet a further object of this invention to provide yogurt or other food products containing viable organisms which are storage stable below 20°C and/or at pH 5.5.

In one aspect the invention provides a method of mutating an organism for use in making a fermented food product which comprises:

a) selecting an organism suitable for use in making a fermented food product;

b) removing the gene which codes for an enzyme involved in the metabolism of a substrate to a desired product from the organism of step a);

c) inserting the gene of b) in a vector;

d) performing mutations on a plurality of vectors of step c);

e) selecting those vectors of step d) in which said gene is mutated so that when a selected vector is used to transform an organism, expression of the selected gene produces a variant enzyme which exhibits decreased activity at a rate of at least 20% less than the enzyme produced by the organism found in nature from which the variant enzyme is derived, under the storage conditions of the fermented food product; but where the variant enzyme retains at least 90% of its activity at the production conditions of the fermented food products when compared with the enzyme produced by the organism found in nature from which the variant enzyme is derived;

f) inserting and expressing a selected gene coding for the variant enzyme of step (e) into an organism suitable for the production of the fermented food product; and

g) culturing the organism into which the gene has been inserted.

Preferably the organism used in step (e) is one from which the corresponding native gene sequence has been removed.

In a second aspect the invention provides a method for making a fermented food product comprising performing the method of the first aspect, and wherein said step of culturing the organism employs as a substrate source an unfermented food product selected so that the culturing leads to formation of the desired fermented food product. The food product may be a fermented dairy product, particularly yogurt.

In a third aspect the invention provides a method of making a gene coding for a variant of an enzyme, comprising performing steps (a) to (e) as set forth above.

In a fourth aspect the invention provides a method of making a plasmid containing a gene coding for a variant of an enzyme, comprising performing steps (a) to (e) as set forth above, wherein the vector is a plasmid.

In a fifth aspect the invention provides a fermented food comprising a viable lactic acid producing organism, said organism having inserted into the genome or inserted by use of a replicable plasmid, a gene encoding for the production of an enzyme active in the lactose to lactic acid pathway of the organism and having a mutation in the amino acid sequence, wherein the production rate of lactic acid by the enzyme in the organism is at least 90% of that of an enzyme without the mutation under the fermentation conditions of the food, and the production rate of lactic acid by the enzyme in the organism is at least 20% less than that of an enzyme without the mutation when the fermented food is at a temperature of 20° or less or a pH of 5.5 or less.

In a sixth aspect the invention provides a fermented dairy product produced by means of a variant L. bulgaricus organism which produces a non-naturally occurring variant enzyme active in the lactose to lactic acid pathway of the organism and differing from the wild-type enzyme in that at a temperature of 20° or less or a pH of 5.5 or less the activity of the enzym is reduced so that lactic acid production is at least 20% less than would be the case for a wild-type organism; whereas under the production conditions of the food, the rate of production of lactic acid is at least 90% of that of a wild-type organism; said dairy product containing said variant organism in viable state.

Fermented food products are those foods in which an active bacterial culture remains in a food and wherein the active bacterial culture has metabolized a specific substrate to produce a desired taste, pH or lowered pH to preserve the food. As discussed above, such foods and the organisms to produce them, are

3

widely know, for example: vegetable products may use <u>Pseudomonas</u>, <u>Flavobacterium</u>, <u>Aerobacter</u>, <u>Bacillus</u>, <u>Leuconostoc</u>, and <u>Lactobacillus</u>; fermented fish products may use <u>Leuconostoc mesenteroides</u>, <u>Lactobacillus brevis</u>, <u>Pediococcus cerevisea</u>, <u>Lactobacillus plantarium</u>, and <u>Bacillus</u> species; fermented seed may use <u>Bacillus natto</u>; fermented starch-rich materials may use <u>Corynebacterium</u> species; fermented meat products may use <u>Lactobacillus</u> species; fermented melons may use <u>Corynebacterium</u> species, <u>Geotrichum candidum</u>, <u>Leuconistoc</u> species; fermented fruit juices may use <u>Zymononas</u> species; soy sauce uses <u>Asperqillus</u> species; and wine and beer use <u>Saacharomyces</u> species. As other species of bacteria, yeast and fungi are developed for food, it is clear that they would be covered also and are contemplated within the scope of the invention. A preferred fermented food product is the fermented dairy products, especially yogurt. The desired substrate as used herein is the substrate the selected organism will metabolize in the metabolic pathway of the final compund which produces a desired taste or pH. So, for example, when using a <u>L. bulgaricus</u> organism to produce yogurt, the desired substrate is lactose or an intermediate which is metabolized to lactic acid.

As used herein the normal storage conditions refers to the physical conditions such as pH or temperature under which one would hold the food while it was awaiting consumption.

Previous attempts to affect the storage performance of organisms for making fermented food products, such as <u>L. bulgaricus</u>, without affecting the fermented food-making capabilities have been largely unsuccessful. The effects of classical mutation techniques, e.g., chemical, u.v., on the metabolic pathway have been global; i.e., a decrease in the metabolic rate at storage conditions was accompanied by an unacceptable change in metabolic rate at production conditions or, most importantly, they have been undefined or nontransferable to other starter strains of <u>L. bulgaricus</u>. It has been discovered unexpectedly that when a gene coding for an enzyme in the metabolic pathway is mutated <u>in vitro</u>; i.e., the gene removed from the organism; that chemical mutation of the gene will produce a percentage of variants which exhibit at least a 20% decrease in metabolism below 20°C and/or pH 5.5 or less, yet retain at least at least 90% activity at the production conditions of the fermented food products when expressed in a transformed organism. In a preferred environment the mutants selected have essentially no change in activity of the modified enzyme. The measurement of change is against the original form of the enzyme found in nature. It is, of course, possible to achieve such mutations after several successive generations of gene mutations so that a direct comparison of metabolic rates of successive mutants does not appear to produce the desired change. The change in the desired mutant would still, however, be compared against the form of the organism found in nature. The mutated gene may then be used to transform another similar organism (or other desired organism) to achieve the organism of the invention using methods known in the art.

As used herein <u>L. bulgaricus</u> organisms suitable for making yogurt or other fermented dairy products are widely utilized naturallyoccuring food grade organisms useful in various commercial applications. As described above, <u>L. bulgaricus</u> is used as one of the organisms in the production of yogurt or other fermented dairy products. <u>L.bulgaricus</u> produces a number of enzymes which are responsible for the metabolism of lactose to lactic acid. Most notable is a permease which is responsible for the transport of lactose into the <u>L. bulgaricus</u> organism and $\beta$-galactosidase which metabolizes lactose directly to glucose and galactose. Glucose is further metabolized and other enzymes in this pathway include glucokinase, phosphoglucose isomerase, phosphofructokinase, aldolase, triose phosphate isomerase, phosphoglycerate kinase, phosphoglyceromutase, enolase, pyruvate kinase, and lactate dehydrogenase (Henry R. Mahler and Eugene Cordes, <u>In Biological Chemistry</u>, Harper & Row, New York, New York, 1966). In other organisms, other pathways such as the pentose phosphate or Entner-Doudoroff pathway, among others could be used (Gerhard Gottschalk, "In Bacterial Metabolism", 1979). Permease and $\beta$-galactosidase are especially interesting because in the lactose to lactic acid pathway; they are the only enzymes in this metabolic pathway, which do not appear to be substantially involved in some other metabolic function.

Preferred genes in the lactic acid pathway for mutation are those coding for lactose permease, See The Lactose Transport System of <u>Streptococcus thermophilus</u>: A Hybrid Protein with Homology to the Melibiose Carrier and Enzyme III of PEP-Dependant Phosphotransferase Systems from Genencor attached, B. Poolman, T. Royer, S. Mainzer and B. Schmidt., J. of Bateriol. (in press), <u>Molec. Gen. Genet.</u>, pp. 159, 239-248 (1978); <u>Nature</u>, Vol. 283, pp. 541 - 545, February 7, 1980; and $\beta$-galactosidase (See <u>The Cloning, Expression and Sequencing of the Beta-Galactosidase Gene From Lactobacillus Bulgaricus Into E. Coli</u>, from Genencor presented at the Second Symposium on Lactic Acid Bacteria on September 22 - 25, 1987, Wageningen, The Netherlands by Schmidt, B. et al., J. of Bacteriol. (in press). As used in the specification and claims, vector relates to a gene sequence which includes at least the gene coding for the enzyme of interest. This form could be linear DNA, a plasmid of some type, or the like. The vector may be of the replicable type, a type which is incorporated into the chromosome for replication or a gene sequence which must be combined with some other gene sequence or shuttle mechanism for incorporation into an organism in a replicable form.

When producing the mutant organism of the invention, the vector may be mutated in vitro by any number of chemical mutagens. For instance, the single and double stranded chemical mutagenesis procedures illustrated in the examples. Other methods of mutation include alpha thiol misincorporation, cassette mutagenesis or other methods of site specific mutagenesis. Applicant has noted that approximately 1 in 1000 - 5000 transformants appear to achieve the desired attributes of the invention. The results are reproducible and just a matter of screening a number of mutants. In order to test such a large number of variants in a reasonable time period, the mutated gene can be reinserted in a replicable and expressible form, either in a plasmid or integrated into the chromosome of an organism and tested. One satisfactory preferred method (see also examples) involves construction of a plasmid using the $\beta$-galactosidase gene, performing the mutagenesis in vitro on the plasmid and then transforming a relatively easily replicable organism like E. coli (or L. bulgaricus or L. casei). The transformed organisms can be screened to see if reaction conditions are such that they meet the desired conditions. Where the clone normally produces a similar enzyme to that being expressed, it is desirable that that gene for the similar enzyme be removed from the host. In this example, the naturally occurring $\beta$-galactosidase gene could be removed from the host L. bulgaricus.

Primarily, there will be two types of preferred mutants screened for: temperature sensitive conditional (C$^s$) and pH sensitive conditional (PH$^s$), although other parameters are possible. Some combination of types of mutants may obviously also be desirable. For example, a temperature conditional mutant for use in the production of yogurt will be one which exhibits a decrease in metabolism of lactose below 20°C, yet only exhibit a 10% or less decrease in activity at processing temperature; i.e. 35°C - 45°C. Likewise, a pH conditional mutant for use in the production of yogurt will exhibit a decrease in activity of at least 20% at pH's of 5.5 or less and retain at least at least 90% activity at the beginning processing pH of the fermented food product compared to wild type enzymes.

Once the proper gene sequence is selected from the variant enzymes, it can be reincorporated into the organism of choice in such a manner that it replicates; i.e., plasmid or homologous genome integration, preferably, as suggested above, with the corresponding native gene sequence previously removed. The transformed organism of the invention can then be used to produce a fermented food product with improved shelf life using conventional techniques (without introducing any foreign DNA since only the mutated enzyme will be replaced in the organism). In a preferred embodiment the fermented food product made will have a longer shelf life, better taste and texture than the fermented food product made without this invention after extended storage.

The following examples are representative of the invention. One skilled in the art will see that the success rate is such that the experiments are easily reproduced and as such, no deposits have made of organisms or genes. One skilled in the art could devise other screens or use other direct mutagenesis techniques without undue experimentation.

## DESCRIPTION OF THE PREFERRED EMBODIMENTS

CHEMICAL MUTAGENESIS OF $\beta$-GALACTOSIDASE GENE

1. Double-Stranded Method

Plasmid Construction. Two BamHI fragments were deleted from the pKK223-3 vector (Pharmacia Molecular Biologicals, Piscataway, New Jersey 08854) that carries the L. bulgaricus $\beta$-galactosidase gene on the 7 kb insert. The resulting plasmid has the tac promoter (deBoer, H.A. et. al., PNAS, Vol. No. 78, pg. 21, (1983)) removed from the pKK223-3 vector and the $\beta$-galactosidase gene is now on a 4.3 kb HindIII-BamHI fragment.

Mutagenesis. Plasmid DNA (~5 ug) in 10mM Tris-HCl and 1 mM Na$_2$EDTA (pH 7.6) was added to 7 ul of 1 M potassium phosphate (pH 5.2), 20 ul 3 M sodium acetate (pH 4.5) and water for a final volume of 0.1 ml. Then 0.1 ml of 2 M hydroxylamine (HA) in ethylene glycol was added to the DNA solution. The reaction was incubated at 65 or 75°C with 20 ul aliquots removed at various times from 0 to 10 minutes. The DNA in the aliquots was immediately precipitated with 2 ul 3 M sodium acetate and 50 ul ethanol. The dried precipitate was dissolved in 30 ul 10 mM Tris-HCl and 1mM Na$_2$EDTA (pH 7.6) and used to transform E. coli JM105, JM108 and JM109 cells (Maniatis, T.; Fritsch, E.F.; Sambrook, J., "Transformation Protocol", In Molecular Cloning: A Laboratory Manual, p. 250, 1983). The transformation mix was plated on Luria Agar plates containing X-gal (5-Bromo-4-chloro-3 indoyl-B-D galactopyranoside, Sigma Chemical Co., St. Louis, Missouri)and 50 ug/ml carbenicillin. Mutants were screened as discussed below.

2. Single-Stranded Method

Plasmid Construction. The β-galactosidase gene on the 4.3 kb HindIII-BamHI fragment was cloned into pUC118. Single-stranded DNA was isolated from this clone after the addition of M13 helper phage (the pUC118 vector has a 470 bp M13 replicon inserted into the NdeI site of pUC18). Unique PstI and XbaI sites were introduced at nucleotides 23 and 441, respectively, using oligonucleotide site directed mutagenesis. Single-stranded DNA from this mutant was used in the chemical mutagenesis described below.

Mutagensis. First, ethylene glycol, 50 ul, was added to 150 ul of 1M sodium acetate containing 1M methoxylamine hydrochloride (MA) (pH 5). Then single-stranded DNA in 10 ul (~5ug) was added and incubated in the dark at 50°C for 45, 60, 90 or 120 minutes. The reaction was stopped by ethanol precipitation and JM105 cells transformed and plated as described above. The transformation mix was plated on X-gal-containing medium and screened as described below.

SELECTION OF TEMPERATURE CONDITIONAL MUTANTS

A nitrocellulose filter is placed on a Luria Agar carbenicillin plate. The transformed E. coli are spread on top and colonies grown overnight at 37°C. Two nitrocellulose replica filters are made from this master. One filter is layered on an X-gal plate at 37°C and β-galactosidase activity is detected by the appearance of blue colonies. The other filter is layered on a Luria Agar carbenicillin plate, grown for about 20 hours at 37°C then placed at 4°C overnight. The filter is lifted off the plate and placed on an X-gal plate at 4°C overnight. Colonies of the invention are those that are blue at 37°C but are white at 4°C.

Second Round Mutagenesis.

Starting with the C$^s$4 variant phenotype which is blue on 37°C X-gal plates and using MA as a mutagen, additional mutants were generated through a second round of mutagenesis.

MUTAGENESIS RESULTS

Approximately 12,000 colonies have been screened using the double- and single-stranded mutagenesis methods. In a typical experiment, 0.1 - 0.2% of the total colonies are white at 37°C and 2-4% are white at 4°C. Colonies that were blue at 37°C and white at 4°C were double checked for the temperature conditional phenotype by replating at the high and low temperatures. Plasmid DNA was isolated from these colonies and used to transform JM105 or JM109 E. coli cells and a consistent temperature conditional β-galactosidase activity was conferred by the plasmids. To localize the region of the mutation, various restriction fragments of the mutant gene were exchanged for wild type fragments. A characterization of 3 of the mutants is given below:

CS1 and CS4: Wild type plasmids and plasmids isolated from this mutant were digested with NcoI and then the 1282 bp NcoI fragments and the vector fragments (carrying the rest of the β-galactosidase gene) were isolated from polyacylamide gels. The mutant 1282 bp fragment was ligated into the wild type vector and the 1282 bp wild type fragment was ligated into the mutant vector. The construction with the 1282 bp NC0I mutant restriction fragment was temperature conditional while the other construction gave a wild type phenotype. Thus, the temperature conditional mutation lies between nucleotides 792 and 2074 (the NcoI fragment) in the structural gene of L. bulgaricus β-galactosidase in this mutant.

CS2: The structural gene for β-galactosidase from wild type and mutant plasmids were removed on a BamHI-XbaI fragment. The wild type structural gene was hooked up to the mutant promoter and the mutant structural gene was hooked up to the wild type promoter. Only the construction containing the mutant structural gene had a temperature conditional phenotype. Thus, the mutation is in the structural part of the gene and in the promoter region in this mutant.

CS3: By using identical methods as described for CS2, the mutation in this mutant was also localized in the structural gene and not in the promoter region.

The following table lists the results of the 27 β-galactosidase mutants obtained in the screen:

PHENOTYPES OF β-GALACTOSIDASE MUTANTS

| Mutant | Plasmid | Mutagen | *Colony color after 24 hours | |
| --- | --- | --- | --- | --- |
| | | | 37°C | 4°C |
| Control (wt) | pKK223-3 or pUC118 | — | blue | blue |
| $C^S$ 1 | pKK223-3 | HA | light blue | white |
| $C^S$ 2 | pUC118 | MA | blue | white |
| $C^S$ 3 | pUC118 | MA | blue | white |
| $C^S$ 4 | pUC118 | MA | blue | light blue |
| $C^S$ 5 | pUC118 | MA | blue | white |
| $C^S$ 6 | pKK223-3 | HA | blue | light blue |
| $C^S$ 7 | pKK223-3 | HA | light blue | light green |
| $C^S$ 8 | pKK223-3 | HA | light blue | light green |
| $C^S$ 11 | pKK223-3 | HA | light blue | light green |
| $C^S$ 12 | pKK223-3 | HA | blue | light blue |
| $C^S$ 13 | pKK223-3 | HA | light blue | light blue |
| $C^S$ 14 | pKK223-3 | HA | light blue | light blue |
| $C^S$ 15 | pKK223-3 | HA | blue | light blue |
| $C^S$ 16 | pKK223-3 | HA | blue | white |
| $C^S$ 18 | pKK223-3 | HA | light blue | white |
| $C^S$ 19 | pKK223-3 | HA | light blue | white |
| $C^S$ 20 | pKK223-3 | HA | blue | light green |
| $C^S$ 21 | pKK223-3 | HA | blue | light green |
| $C^S$ 22 | pKK223-3 | HA | light blue | light blue |
| $C^S$ 23 | pKK223-3 | HA | light blue | light blue |

PHENOTYPES OF ß-GALACTOSIDASE MUTANTS

| Mutant | Plasmid | Mutagen | *Colony color after 24 hours | |
| --- | --- | --- | --- | --- |
| | | | 37°C | 4°C |
| C$^S$ 24 | pKK223-3 | HA | blue | white |
| C$^S$ 25 | pKK223-3 | HA | blue | white |
| C$^S$ 26 | pKK223-3 | HA | blue | white |
| C$^S$ 28 | pKK223-3 | HA | blue | white |
| C$^S$ 29 | pKK223-3 | HA | light blue | light green |
| C$^S$ 31 | pKK223-3 | HA | blue | light green |
| C$^S$ 32 | pKK223-3 | HA | blue | light green |

*ß-galactosidase activity measured on X-gal plates by the degree of blueness exhibited by colonies; i.e., blue = wild-type activity; white = inactive or no ß-galactosidase activity; and green = intermediate activity.

The colonies from all the temperature conditional mutants listed above do turn light to medium blue on X-gal plates after 1-3 weeks at 4°C.

SELECTION OF pH SENSITIVE MUTANTS

Similarly, the same mutations may be screened for pH sensitivity as follows:
Since the controlled fermentation is normally stopped at pH 5.5 or less, it was felt that mutants which began to exhibit the desirable characteristics (decreased rate of acidification) at about pH 5.5 - 6.0 would be useful under production and storage conditions of pH of about 4.5 and below.

A nitrocellulose filter is placed on a Luria Agar carbenicillin plate. The transformed E. coli are spread on top and colonies grown overnight at 37°C. Two nitrocellulose replica filters are made from this master. The cells are lysed by 3 minute chloroform exposure. The control filter is placed in 25ml of a 20mM $MgSO_4$ pH7, 10mM $NaH_2PO_4$, 50mM NaOAc buffer for 15 minutes and transferred to the same buffer containing 0.2 ml 2% X-gal. The other filter is placed in a low pH (5-5.5) 100mM $NaH_2PO_4$, 2mM $MgSO_4$, 50mM NaOAc buffer for 15 minutes. Then the filter is transferred to a low pH buffer containing X-gal. A positive colony is one that is blue at pH 7 and white at pH 4.5 or 5.0.

Deletion of the host $\beta$-galactosidase (or permease) and reincorporation of the mutant gene back into L. bulgaricus can then be done by one skilled in the art. For example, the methods in Journal of Bacteriology, May 1984, pp. 411-418 and Journal of Bacteriology, November 1988, pp. 5102 - 5109 would generate the necessary reincorporation mutant L. bulgaricus organisms.

## RESULTS

## LOW pH$^S$ β-GALACTOSIDASE MUTANTS

Source: Isolated from methoxyl amine mutagenesis of mutant C$^S$4

First screen: performed at pH 5

### PHENOTYPE

| | after 40 minutes | | after 24 hours | |
|---|---|---|---|---|
| MUTANT | pH5 | pH7 | 37$^O$C | 4$^.$C |
| pH$^S$8-1 | white | white | blue | light green white |
| pH$^S$8-2 | white | blue | light green | light blue |
| pH$^S$8-3 | white | blue | blue | light blue |
| pH$^S$8-4 | white | blue | not tested | |
| pH$^S$8-5 | white | blue | blue | blue |
| pH$^S$8-6 | white | blue | blue | blue |
| pH$^S$8-7 | white | blue | blue | blue |
| pH$^S$8-8 | white | blue | light blue | light blue |

CONTROL

| | | | | |
|---|---|---|---|---|
| pKK223-3 | light blue | blue | blue | blue |

Second screen: performed at pH 5.5

9

**PHENOTYPE**

|  | after 30 minutes | | after 24 hours | |
|---|---|---|---|---|
| MUTANT | pH5.5 | pH7 | 37°C | 4°C |
| pH$^S$2-1 | white | blue | light blue | light blue |
| pH$^S$2-2 | white | blue | light blue | blue+white |
| CONTROL |  |  |  |  |
| pKK223-2 | light blue | blue | blue | blue |

PURIFICATION AND CHARACTERIZATION OF MUTANT AND WILD-TYPE $\beta$-GALACTOSIDASE

Mutant and wild-type enzyme could be purified from E. coli by the following protocol:

```
cell lysate
    ↓
high-speed centrifugation
    ↓
desalt
    ↓
octyl sepharose hydrophobic chromatography
    ↓
desalt
    ↓
MonoQ ion exchange chromoatography
    ↓
concentration
    ↓
superose 12 sizing column
    ↓
pure protein
```

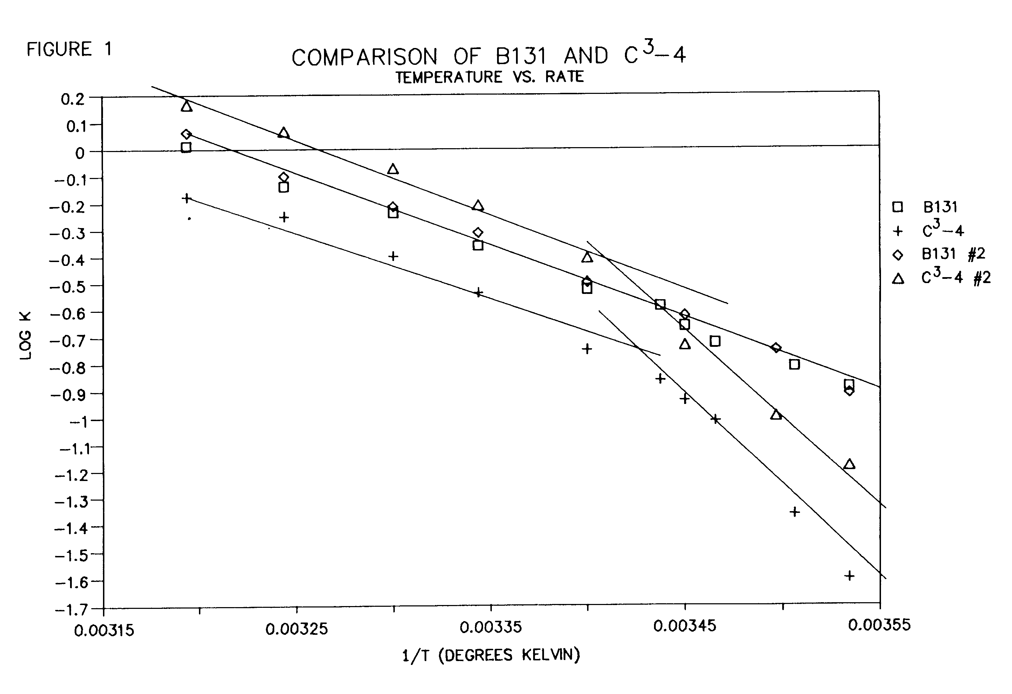

Enzyme purified by this procedure was measured for activity at a variety of temperatures from 10 - 40°C. Figure 1 is a plot of the log of the apparent initial rate constant vs. the reciprocal of the absolute temperature for two concentrations of mutant C$^s$4 and wild-type enzyme. While the slope of the wild-type enzyme is linear over the entire temperature range, the mutant clearly shows a decrease in rate below 18°C. Furthermore, above 20°C the slope for both the wild-type and mutant enzymes are identical. The data clearly indicates that a C$^s$4 enzyme has been produced which is equal in activity to wild-type at production temperatures but reduced in activity at storage temperatures.

**Claims**

1. A method of mutating an organism for use in making a fermented food product which comprises:

a) selecting an organism suitable for use in making a fermented food product;

b) removing the gene which codes for an enzyme involved in the metabolism of a substrate to a desired product from the organism of step a);

c) inserting the gene of b) in a vector;

d) performing mutations on a plurality of vectors of step c);

e) selecting those vectors of step d) in which said gene is mutated so that when a selected vector is used to transform an organism, expression of the selected gene produces a variant enzyme which exhibits decreased activity at a rate of at least 20% less than the enzyme produced by the organism found in nature from which the variant enzyme is derived, under the storage conditions of the fermented food product; but where the variant enzyme retains at least 90% of its activity at the production conditions of the fermented food products when compared with the enzyme produced by the organism found in nature from which the variant enzyme is derived;

f) inserting and expressing a selected gene coding for the variant enzyme of step (e) into an organism suitable for the production of the fermented food product; and

g) culturing the organism into which the gene has been inserted.

2. A method according to claim 1 wherein the organism is a lactic acid producing organism.

3. A method according to claim 2 wherein said enzyme is active in a lactose-to-lactic acid pathway of the organism.

4. A method according to any preceding claim wherein said decreased activity is exhibited in an organism containing the enzyme when at a temperature of below 20°C and/or at a pH $\leq$ 5.5.

5. A method according to any preceding claim wherein the enzyme is a $\beta$-galactosidase or a permease.

6. A method according to any preceding claim wherein the organism is L. bulgaricus.

7. A method according to any preceding claim wherein in step (f) the gene is inserted in a plasmid or with genomic integration, whereby it is replicable.

8. A method according to any preceding claim wherein step (d) employs a mutagenesis process selected from single stranded chemical mutagenesis, double stranded chemical mutagenesis, $\alpha$-thiol misincorporation or cassette mutagenesis.

9. A method according to any preceding claim wherein in step (f) the selected gene is inserted into an organism from which a corresponding native gene sequence has been removed.

10. A method for making a fermented food product comprising performing the method of any preceding claim, wherein said step of culturing the organism employs as a substrate source an unfermented food product selected so that the culturing leads to formation of the desired fermented food product.

11. A method according to claim 10 wherein the fermented food product is a fermented dairy product.

12. A method according to claim 11 wherein the fermented food product is yogurt.

13. A method according to claim 10, 11 or 12 wherein said organism which is cultured to produce the food product is L. bulgaricus.

14. A method of making a gene coding for a variant of an enzyme, comprising performing steps (a) to (e) as set forth in claim 1.

15. A method of making a plasmid containing a gene coding for a variant of an enzyme, comprising performing steps (a) to (e) as set forth in claim 1, wherein the vector is a plasmid.

16. A method of making a variant of an enzyme, comprising performing the method of claim 1 and then isolating the enzyme from the organism.

**17.** A method according to claim 14, 15 or 16 wherein the enzyme is $\beta$-galactosidase or permease.

**18.** A fermented food comprising a viable lactic acid producing organism, said organism having inserted into the genome or inserted by use of a replicable plasmid, a gene encoding for the production of an enzyme active in the lactose to lactic acid pathway of the organism and having a mutation in the amino acid sequence, wherein the production rate of lactic acid by the enzyme in the organism is at least 90% of that of an enzyme without the mutation under the fermentation conditions of the food, and the production rate of lactic acid by the enzyme in the organism is at least 20% less than that of an enzyme without the mutation when the fermented food is at a temperature of 20° or less or a pH of 5.5 or less.

**19.** A fermented food according to claim 18 which is a fermented dairy product.

**20.** A fermented dairy product produced by means of a variant <u>L. bulgaricus</u> organism which produces a nonnaturally occurring variant enzyme active in the lactose to lactic acid pathway of the organism and differing from the wild-type enzyme in that at a temperature of 20° or less or a pH of 5.5 or less the activity of the enzyme is reduced so that lactic acid production is at least 20% less than would be the case for a wild-type organism; whereas under the production conditions of the food, the rate of production of lactic acid is at least 90% of that of a wild-type organism; said dairy product containing said variant organism in viable state.

**Patentansprüche**

**1.** Verfahren zum Mutieren eines Organismus zur Verwendung bei der Herstellung eines fermentierten bzw. vergorenen Nahrungsmittelerzeugnisses, welches Verfahren umfaßt:
a) Auswählen eines Organismus, der sich zur Verwendung bei der Herstellung eines fermentierten Nahrungsmittelerzeugnisses eignet;
b) Entfernen des Gens, das für ein Enzym kodiert, das im Metabolismus eines Substrats zu einem erwünschten Produkt aus dem Organismus von Schritt a) beteiligt ist;
c) Einsetzen des Gens von b) in einen Vektor;
d) Durchführen von Mutationen auf einer Vielzahl von Vektoren von Schritt c);
e) Auswählen der Vektoren von Schritt d), in denen das genannte Gen mutiert ist, sodaß bei Verwendung eines ausgewählten Vektors zum Transformieren eines Organismus die Expression des ausgewählten Gens ein Variantenenzym erzeugt, das unter den Lagerbedingungen des fermentierten Nahrungsmittelerzeugnisses eine verminderte Aktivität mit einer Rate von zumindest 20% weniger als das Enzym aufweist, das durch den in der Natur vorkommenden Organismus erzeugt wird, von dem das Variantenenzym abgeleitet ist; doch wobei das Variantenenzym zumindest 90% seiner Aktivität unter den Produktionsbedingungen der fermentierten Nahrungsmittelerzeugnisse im Vergleich zum Enzym beibehält, das durch den in der Natur vorkommenden Organismus erzeugt wird, von dem das Variantenenzym abgeleitet ist;
f) Einsetzen und Exprimieren eines ausgewählten Gens, das für das Variantenenzym von Schritt (e) kodiert, in einen Organismus, der sich zur Herstellung des fermentierten Nahrungsmittelerzeugnisses eignet; und
g) Züchten des Organismus, in den das Gen eingesetzt wurde.

**2.** Verfahren nach Anspruch 1, worin der Organismus ein milchsäureproduzierender Organismus ist.

**3.** Verfahren nach Anspruch 2, worin das genannte Enzym in einem Lactose-zu Milchsäure-Weg des Organismus aktiv ist.

**4.** Verfahren nach einem der vorhergehenden Ansprüche, worin die genannte verminderte Aktivität in einem Organismus auftritt, der bei einer Temperatur von weniger als 20°C und/oder mit einem pH-Wert von $\leq$.5,5 das Enzym enthält.

**5.** Verfahren nach einem der vorhergehenden Ansprüche, worin das Enzym eine $\beta$-Galactosidase oder eine Permease ist.

**6.** Verfahren nach einem der vorhergehenden Ansprüche, worin der Organismus L.bulgaricus ist.

**7.** Verfahren nach einem der vorhergehenden Ansprüche, worin in Schritt (f) das Gen in ein Plasmid oder durch genomische Integration eingefügt wird, wodurch es replizierbar ist.

**8.** Verfahren nach einem der vorhergehenden Ansprüche, worin in Schritt (d) einen Mutageneseprozeß angewendet wird der aus chemischer Einzelstrang-Mutagenese, Doppelstrang -Mutagenese, α-Thiol-fehleinbau oder Kassettenmutagenese ausgewählt ist.

**9.** Verfahren nach einem der vorhergehenden Ansprüche, worin in Schritt (f) das ausgewählte Gen in einen Organismus eingesetzt wird, aus dem eine entsprechende native Gensequenz entfernt worden war.

**10.** Verfahren zum Herstellen einer fermentierten Nahrungsmittelerzeugnisses unter Durchführung des Verfahrens nach einem der vorhergehenden Ansprüche, worin im genannten Schritt des Züchtens des Organismus als Substratquelle ein nicht fermentiertes Nahrungsmittelerzeugnis eingesetzt wird, sodaß das Züchten zur Bildung des gewünschten fermentierten Nahrungsmittelerzeugnisses führt.

**11.** Verfahren nach Anspruch 10, worin das fermentierte Nahrungsmittelerzeugnis ein fermentiertes Molkereiprodukt ist .

**12.** Verfahren nach Anspruch 11, worin das fermentierte Nahrungsmittelerzeugnis Joghurt ist.

**13.** Verfahren nach Anspruch 10, 11 oder 12, worin genannter Organismus, der zur Herstellung des Nahrungsmittelerzeugnisses gezüchtet wird, L.bulgaricus ist.

**14.** Verfahren zur Herstellung eines Gens, das für eine Variante eines Enzyms kodiert, das die Durchführung der in Anspruch 1 dargelegten Schritte (a) bis (e) umfaßt.

**15.** Verfahren zur Herstellung eines Plasmids, das ein Gen enthält, das für eine Variante eines Enzyms kodiert, das das Durchführen der in Anspruch 1 dargelegten Schritte (a) bis (e) umfaßt, worin der Vektor ein Plasmid ist.

**16.** Verfahren zur Herstellung einer Variante eines Enzyms, das das Durchführen des Verfahrens nach Anspruch 1 und das anschließende Isolieren des Enzyms aus dem Organismus umfaßt.

**17.** Verfahren nach Anspruch 14, 15 oder 16, worin das Enzym β-Glactosidase oder Permease ist.

**18.** Fermentiertes Nahrungsmittel umfassend einen lebensfähigen milchsäureproduzierenden Organismus, wobei genannter Organismus ein in das Genom oder mittels eines replizierbaren Plasmids eingesetztes Gen aufweist, wobei das Gen für die Herstellung eines Enzyms kodiert, das im Lactose-zu-Milchsäure-Weg des Organismus aktiv ist und eine Mutation in der Aminosäuresequenz aufweist, wobei die Produktionsrate von Milchsäure durch das Enzym im Organismus zumindest 90% jener eines Enzyms ohne die Mutation unter den Fermentierungsbedingungen des Nahrungsmittels beträgt, und die Produktionsrate von Milchsäure durch das Enzym im Organismus zumindest 20% niedriger als jene eines Enzyms ohne die Mutation ist, wenn das fermentierte Nahrungsmittel eine Temperatur von 20 °C oder darunter oder einen pH-Wert von 5,5 oder weniger aufweist.

**19.** Fermentiertes Molkereiprodukt nach Anspruch 18, das ein fermentiertes Molkereiprodukt ist.

**20.** Fermentiertes Molkereiprodukt, das mittels einer Variante eines L.bulgaricus Organismus hergestellt ist, der ein nicht in der Natur vorkommendes Variantenenzym erzeugt, das im Lactose-zu-Milchsäure-Weg des Organismus aktiv ist und sich insofern vom Enzym des wilden Typs unterscheidet, als die Aktivität des Enzyms bei einer Temperatur von 20 °C oder darunter oder einem pH-Wert von 5,5 oder weniger vermindert ist, sodaß die Milchsäureproduktion von zumindest 20% geringer ist als bei einem Organismus des wilden Typs; während unter den Herstellungsbedingungen des Nahrungsmittels die Produktionsrate von Milchsäure zumindest 90% jener eines Organismus des wilden Typs ist; wobei das genannte Molkereiprodukt genannten Variantenorganismus in lebensfähigem Zustand enthält.

**Revendications**

1. Méthode de mutation d'un organisme pour utilisation dans la fabrication d'un produit alimentaire fermenté qui comprend :

   a) sélection d'un organisme approprié pour utilisation dans la fabrication d'un produit alimentaire fermenté,

   b) élimination du gène qui code un enzyme appliqué dans le métabolisme d'un substrat à un produit désiré provenant de l'organisme de l'étape a) ;

   c) insertion du gène de b) dans un vecteur ;

   d) mise en oeuvre des mutations sur une pluralité de vecteurs de l'étape c) ;

   e) sélection de ces vecteurs de l'étape d) dans lesquels ledit gène est muté de sorte que lorsqu'un vecteur sélectionné est utilisé pour transformer un organisme, une expression du gène choisi produit un enzyme variant qui montre une activité diminuée à un taux d'au moins 20% inférieure à celle de l'enzyme produit par l'organisme trouvé dans la nature dont l'enzyme variant est dérivé, dans les conditions de stockage du produit alimentaire fermenté ; mais où l'enzyme variant garde au moins 90% de son activité dans les conditions de production des produits alimentaires fermentés lorsque comparée avec l'enzyme produit par l'organisme trouvé dans la nature dont l'enzyme variant est dérivé ;

   f) insertion et expression d'un gène sélectionné codant l'enzyme variant de l'étape (e) en un organisme approprié pour la production du produit alimentaire fermenté ; et

   g) culture de l'organisme dans lequel le gène a été inséré.

2. Méthode selon la revendication 1 dans laquelle l'organisme est un organisme produisant un acide lactique.

3. Méthode selon la revendication 2 dans laquelle ledit enzyme est actif dans un schéma de transformation de lactose en acide lactique de l'organisme.

4. Méthode selon l'une quelconque des revendications précédentes, dans laquelle ladite activité diminuée est montrée dans un organisme contenant l'enzyme lorsqu'à une température en dessous de 20°C et/ou à un pH inférieur ou égal à 5,5.

5. Méthode selon l'une quelconque des revendications précédentes, dans laquelle l'enzyme est une *β*-galactosidase ou une perméase.

6. Méthode selon l'une quelconque des revendications précédentes, dans laquelle l'organisme est L. bulgaricus.

7. Méthode selon l'une quelconque des revendications précédentes, dans laquelle à l'étape (f) le gène est inséré dans un plasmide ou avec une intégration génomique, ce par quoi il est réplicable.

8. Méthode selon l'une quelconque des revendications précédentes, dans laquelle l'étape (d) emploie un procédé de mutagenèse choisi parmi la mutagenèse chimique à brin unique, une mutagenèse chimique à double brins, une incorporation *α*-thiol ou une mutagenèse de cassette.

9. Méthode selon l'une quelconque des revendications précédentes, dans laquelle dans l'étape (f) le gène choisi est inséré dans un organisme dont une séquence de gène native correspondante a été enlevée.

10. Méthode pour fabriquer un produit alimentaire fermenté comprenant la mise en oeuvre de la méthode de l'une quelconque des revendications précédentes, dans laquelle ladite étape de culture de l'organisme emploie en tant que source substrat un produit alimentaire non fermenté choisi de sorte que la culture mène à la formation du produit alimentaire fermenté désiré.

11. Méthode selon la revendication 10, dans laquelle le produit alimentaire fermenté est un produit laitier fermenté.

12. Méthode selon la revendication 11, dans laquelle le produit alimentaire fermenté est du yoghourt.

**13.** Méthode selon la revendication 10, 11 ou 12, dans laquelle ledit organisme qui est cultivé pour produire le produit alimentaire est L. bulgaricus.

**14.** Méthode de fabrication d'un gène codant variant d'un enzyme, comprenant la mise en oeuvre des étapes (a) à (e) décrites en revendication 1.

**15.** Méthode de fabrication d'un plasmide contenant un gène codant variant d'un enzyme, comprenant la mise en oeuvre des étapes (a) à (e) décrites en revendication 1, dans lesquelles le vecteur est un plasmide.

**16.** Méthode de fabrication d'un variant d'un enzyme, comprenant la mise en oeuvre de la méthode de la revendication 1 et ensuite l'isolement de l'enzyme de l'organisme.

**17.** Méthode selon la revendication 14, 15 ou 16, dans laquelle l'enzyme est une $\beta$-galactosidase ou une perméase.

**18.** Produit alimentaire fermenté comprenant un organisme viable produisant de l'acide lactique, ledit organisme ayant inséré dans le génome ou inséré par utilisation d'un plasmide réplicable, un gène encodant pour la production d'un enzyme actif dans le schéma de transformation du lactose en acide lactique de l'organisme et ayant une mutation dans la séquence acide aminé, dans laquelle le taux de production d'acide lactique par l'enzyme dans l'organisme est d'au moins 90% de celle d'un enzyme sans la mutation dans des conditions de fermentation de la nourriture, et le taux de production d'acide lactique par l'enzyme de l'organisme est d'au moins 20% inférieur à celui d'un enzyme sans la mutation lorsque le produit alimentaire fermenté est à une température de 20° ou moins ou à un pH de 5,5 ou moins.

**19.** Produit alimentaire fermenté selon la revendication 18 qui est un produit laitier fermenté.

**20.** Produit laitier fermenté produit au moyen d'un organisme variant L. bulgaricus qui produit un enzyme variant se produisant naturellement actif dans le schéma de transformation du lactose en acide lactique de l'organisme et différent de l'enzyme de type sauvage en ce qu'à une température de 20° ou moins ou à un pH de 5,5 ou moins, l'activité de l'enzyme est réduite de sorte que la production d'acide lactique est d'au moins 20% inférieure à celle qui pourrait être le cas pour un organisme de type sauvage ; tandis que dans les conditions de production de l'aliment, le taux de production d'acide lactique est d'au moins 90% celui d'un organisme de type sauvage ; ledit produit laitier contenant ledit organisme variant à l'état viable.

FIGURE 1

COMPARISON OF B131 AND C$^3$-4
TEMPERATURE VS. RATE

LOG K

1/T (DEGREES KELVIN)

□  B131
+  C$^3$-4
◇  B131 #2
△  C$^3$-4 #2

EP 0 402 450 B1